# EUROPEAN PATENT SPECIFICATION

(11) **EP 4 159 265 B1**
(45) Date of publication and mention of the grant of the patent: **04.12.2024**
(21) Application number: 20938313.2
(22) Date of filing: 12.10.2020
(51) Int. Cl.: A61M 25/14, A61M 25/00, B32B 27/32, B32B 5/02, B32B 27/36, B32B 27/40, B32B 27/34, B32B 7/02, B32B 27/28, B32B 27/12, B32B 15/02, B32B 27/08, B32B 25/14, B32B 15/08, B32B 15/09, B32B 25/12, B32B 25/10, B32B 15/14, B32B 15/18, B32B 15/085, B32B 7/022, A61M 25/01

(54) **MICROCATHETER HAVING DOUBLE CAVITIES**
MIKROKATHETER MIT DOPPELTEN HOHLRÄUMEN
MICROCATHÉTER À DOUBLES CAVITÉS

(30) Priority: 27.05.2020 CN 202010463479
(43) Date of publication of application: 05.04.2023
(73) Proprietor: Insight Lifetech Co., Ltd., Shenzhen, Guangdong 518101 (CN)
(72) Inventor: ZHANG, Pengtao, Shenzhen, Guangdong 518101 (CN); WANG, Yuxiang, Shenzhen, Guangdong 518101 (CN); FENG, Guanmei, Shenzhen, Guangdong 518101 (CN)
(74) Representative: Isern Patentes y Marcas S.L.
(86) International application number: PCT/CN2020/120502
(87) International publication number: WO 2021/238018

(56) References cited:
- EP-A1- 2 944 344
- WO-A1-2018/008272
- WO-A1-2018/083905
- CN-A- 104 161 548
- CN-A- 108 514 677
- CN-A- 110 575 605
- CN-A- 111 544 744
- CN-U- 204 364 615
- CN-U- 205 181 966
- CN-U- 209 270 600
- US-A1- 2005 234 427
- US-A1- 2011 144 581

## Description

### BACKGROUND OF INVENTION

### Field of Invention

The present disclosure relates to a dual lumen microcatheter.

### Description of Related Art

Along with the economic development of society, the national lifestyle has undergone profound changes, especially featured by the aging of the population and the acceleration of urbanization, the prevalence and risks of cardiovascular diseases in China are prominent, resulting in increasing in the number of patients with cardiovascular diseases. Currently there are about 2.2 million patients with chronic total occlusion of the coronary artery in China.

Chronic total occlusion(CTO) of the coronary artery refers to a lesion in which the coronary artery is 100% occluded for more than 3 months, which requires a guide wire to cross the CTO lesion for treatment. To improve the success rate for the guide wire to cross the lesion, generally, a microcatheter needs to be used cooperating with the guide wire. Once the guide wire first reaches the CTO lesion, the microcatheter is introduced into the coronary artery from a guide catheter and moves along the guide wire; after the microcatheter reaches the CTO lesion, the guide wire attemptstocross the CTO lesion. In comparison with the conventional coronary intervention method, since the distance between the microcatheter and the CTO lesion is much smaller than the distance between the guide catheter and the CTO lesion, stronger support may be given to the guide wirewhich facilitates the guide wire to cross the CTO lesion. In addition, since the microcatheter has small sizewhichallows the microcatheter to be advanced alternately with the guide wire during the opening of the CTO lesion, providing continuous additional support for the guide wire to cross the CTO lesion.

However, as bifurcated lesions, or lesions at blood vessel bifurcations are present, the guide wire may enter a false lumen and create a path into the false lumen. As single lumen microcatheter is used, if the guide wire is withdrawn from the false lumen or a second guide wire is used directly to try again crossing the lesion into a true lumen, the guide wire tends to enter the false lumen by inertia along the old path. However, a dual lumen microcatheter is used with one of the guide wires entering the false lumen and the path to the false lumen being blocked by the dual lumen microcatheter, the other guide wire may try to cross the lesion through the other guide wire inner lumen of the dual lumen microcatheter into the true lumen. Since the path to the false lumen is blocked by the dual lumen microcatheter and the first guide wire, the possibility of entering the true lumen of the second guide wire is to be increased very much.

In the prior art, when pressed by an operator towards a distal end, the catheter may be bentinhalfway. A lumen transition part of the dual lumen microcatheter is particularly a location where stress concentrates and fractureoccurmost likely,becausethere is significant difference in flexibilityin the connection part of a first catheter body and a second catheter body of the dual lumen microcatheter,whilefracture usually occur at the place with significant different flexibility. An example of a known dual lumen catheter is provided in WO2018083905 A1.

### SUMMARY OF INVENTION

In view of the above, it is an object of the present disclosure to provide a dual lumen microcatheter, with good push performance, flexibility, as well as excellent tensile resistance and bending resistance. The invention provide a dual lumen catheter according to claim 1.

The present disclosure provides also further examples useful for better understanding of the invention. One example comprises a dual lumen microcatheter, the microcatheter includes a first catheter body having a proximal-end segment, a transition segment, and a distal-end segment connected to each other in sequence, and a second catheter body engaged to a periphery of the first catheter body, the second catheter body has a connecting segmentengaged to the periphery of the first catheter body and tip segmentof taper shapeengagedto the connecting segment; the first catheter body has a first guide wire inner lumen slidably receiving a guide wire, and in the first catheter body, the proximal-end segment has a first inner layer forming the first guide wire inner lumen, a spring layer wound on the first inner layer in winding manner and arranged along a length of the first inner layer, a woven layer provided on an outer side of the spring layer, and a first type polymer layer covering an outer side of the woven layer; the transition segment has the first inner layer extending from the proximal-end segment, a woven layer extending from the proximal-end segment and partially covering an outer side of the first inner layer, and the first type polymer layer extending from the proximal-end segment and covering the outer side of the woven layer; the distal-end segment has the first inner layer extending from the transition segment and the first type polymer layer extending from the transition segment and covering the outer side of the first inner layer; the second catheter body has a second guide wire inner lumen slidably receiving a guide wire; the second catheter body has a second inner layer forming the second guide wire inner lumen and a second type polymer layer covering an outer side of the second inner layer; the connecting segment of the second catheter body is at least partially connected to the transition segment of the first catheter body, and the spring layer does not extend to the transition segment and the distal-end segment.

In the dual lumen microcatheter according to the present disclosure, the guide wire may move through the first guide wire inner lumen of the first catheter body and may move through the second guide wire inner lumen of the second catheter body; the first catheter body includes the proximal-end segment, the transition segment, and the distal-end segment, the proximal-end segment has the inner layer, the spring layer, the woven layer, and the polymer layer, the transition segment has the inner layer, the woven layer, and the polymer layer partially covering the inner layer, and the distal-end segment and the second catheter body have the inner layer and the polymer layer; the second catheter body has the connecting segment and the tip segment. In this case, tip segmentof taper shape can facilitate the advancement of the microcatheter, and the inner layer, the spring layer, the woven layer and the polymer layer of the catheter body are arranged orderly from inside out, hence the catheter is facilitated to be advanced; a joint of the first catheter body and the second catheter body has good flexibility and can form a proper transition with the proximal-end segment. As such, a dual lumen microcatheter that is facilitated to be advanced, strong in compliance, and excellent in both tensile resistance and bending resistance is provided.

In addition, in the microcatheter according to the present disclosure, optionally, in the first catheter body, pliability of the proximal-end segment, flexibility of the transition segment, and pliability of the distal-end segment decrease gradually, and in the second catheter body, pliability of the connecting segment and pliability of the tip segment decrease gradually. Thereby it is able to gradually change the pliability of the microcatheter to mitigate the risk of fracture, resulting in improving the flexibility of the microcatheter as a whole.

In addition, in the microcatheter according to the present disclosure, optionally, an outer diameter of the transition segment decreases gradually from the proximal-end segment to the distal-end segment, and the pliability of the transition segment decreases gradually. Thereby, the passage of the microcatheter in the blood vessel is facilitated.

In addition, in the microcatheter of the present disclosure, optionally, the first type polymer layer includes a first polymer layer disposed at the proximal-end segment and having a first hardness, a second polymer layer disposed at the transition segment and having a secondhardness, and a third polymer layer disposed at the distal-end segment and having a third hardness, and a fourth polymer layer connected to the third polymer layer and having a fourth hardness; the second type polymer layer includes a fifth polymer layer having a fifth hardness, a sixth polymer layer connected to the 5th polymer layer and having a sixth hardness, a seventh polymer layer connected to the sixth polymer layer and having a seventh hardness, an eighth polymer layer connected to the seventh polymer layer and having an eighth hardness, and a nineth polymer layer having a nineth hardness. In this case, the first type polymer layer and the second type polymer layer may be formed witha plurality of polymer layers combined respectively, and different polymer layers may have different hardness. Thereby, a suitable hardness may be selected according to different positions where the different polymer layers are located, further improving the bending resistance of the microcatheter with flexibility ensured.

In addition, in the microcatheter of the present disclosure, optionally, the first hardness is greater than the fourth hardness, the third hardness is equal to the sixth hardness, the fourth hardness is equal to the seventh hardness, and the fifth hardness is greater than the nineth hardness. Thereby, the microcatheter can be enabled to have good flexibility to improve the bending resistance and tensile resistance.

In addition, in the microcatheter according to the present disclosure, optionally, the second hardness, the first hardness, the fifth hardness, the third hardness, the fourth hardness, the sixth hardness, the seventh hardness, the eighth hardness, and the nineth hardness decrease in sequence gradually. Thereby, the hardness of the microcatheter is able to be changed gradually through gradual decrease in hardnessand the microcatheter has goodflexibility.

In addition, in the microcatheter according to the present disclosure, optionally, the second hardness, the first hardness, the fifth hardness, the third hardness, the fourth hardness, the eighth hardness, and the nineth hardness decrease in sequence gradually. In this case, in the first catheter body and the second catheter body, some of the polymer layers may have the same hardness. Thereby, the flexibility difference of polymer layers can be reduced to improve the bending resistance.

In addition, in the microcatheter of the present disclosure, optionally, the second hardness is greater than the 1st hardness, the first hardness is greater than the fifth hardness, the fifth hardness is greater than the third hardness, the third hardness is equal to the sixth hardness and is greater than the fourth hardness, the fourth hardness is equal to the seventh hardness and is greater than the eighth hardness, and the eighth hardness is greater than the nineth hardness.

In addition, in the microcatheter according to the present disclosure, optionally, at a connection part of the first catheter body and the connecting segment, the first type polymer layer further wraps and covers the connecting segment such that the first type polymer layer covers the second type polymer layer. Thereby,the connectivity of the first catheter body and the second catheter body can be improved, resulting in improving the stability of the entire microcatheter.

In addition, in the microcatheter according to the present disclosure, optionally, the spring layer is formed with a wire spirally wound on the first inner layer in winding manner, and the woven layer is formed with a braid wire woven on the outer side of the spring layer. In this case, even if a continuous torque is input from the outside, the spring layer can be protected from falling apart by the woven layer on the outer side thereof, thereby the reliability of the microcatheter can be improved.

In addition, in the microcatheter of the present disclosure, optionally, the first catheter body does not contact the tip segment of the second catheter body. In this case, the distal-end segment of the first catheter body can be connected to the connecting segment of the second catheter body without contacting the tip segment, thereby the tip segment can further extend in a direction parallel to the distal-end segment.

In addition, in the microcatheter of the present disclosure, optionally, the first type polymer layer includes a first polymer layer disposed at the proximal-end segment and having a first elastic modulus, a second polymer layer disposed at the transition segment and having a second elastic modulus, a third polymer layer disposed at the distal-end segment and having a 3rd elastic modulus, and a fourth polymer layer connected to the third polymer layer and having a 4th elastic modulus; the second type polymer layer includes a fifth polymer layer having a fifth elastic modulus, a sixth polymer layer connected to the fifth polymer layer and having a sixth elastic modulus, a seventh polymer layer connected to the sixth polymer layer and has a seventh elastic modulus, an eighth polymer layer connected to the seventh polymer layer and has an eighth elastic modulus, and a nineth polymer layer having a nineth elastic modulus. In this case, the first type polymer layer and the second type polymer layer may be formed with a plurality of polymer layers having different elastic modulus combined. Thereby, a suitable elastic modulus can be selected according to different positions where the different polymer layers are located, the bending resistance of the microcatheter can be further improved with the flexibility ensured.

In addition, in the microcatheter of the present disclosure, optionally, the first elastic modulus is greater than the fourth elastic modulus, the third elastic modulus is equal to the sixth elastic modulus, the fourth elastic modulus is equal to the seventh elastic modulus, and the fifth elastic modulus is greater than the nineth elastic modulus. Thereby, the microcatheter can be enabled to have good flexibility, resulting in improving the bending resistance and tensile resistance.

In addition, in the microcatheter according to the present disclosure, optionally, the second elastic modulus, the first elastic modulus, the fifth elastic modulus, the third elastic modulus, the fourth elastic modulus, the sixth elastic modulus, the seventh elastic modulus, the eighth elastic modulus, and the nineth elastic modulus decrease in sequence gradually. Thereby, the elastic modulus of the microcatheter can be changed gradually through a gradual decrease in the elastic modulus withgoodflexibility ensured.

In addition, in the microcatheter according to the present disclosure, optionally, the second elastic modulus, the first elastic modulus, the fifth elastic modulus, the third elastic modulus, the fourth elastic modulus, the eighth elastic modulus, and the nineth elastic modulus decrease in sequence gradually. In this case, in the first catheter body and the second catheter body, some of the polymer layers may have the same elastic modulus. Thereby, the flexibility difference of the polymer layers may be reduced to improve the bending resistance.

In addition, in the microcatheter of the present disclosure, optionally, the second elastic modulus is greater than the first elastic modulus, the first elastic modulus is greater than the fifth elastic modulus, the fifth elastic modulus is greater than the third elastic modulus, the third elastic modulus is equal to the sixth elastic modulus and greater than the fourth elastic modulus, the fourth elastic modulus is equal to the seventh elastic modulus and greater than the eighth elastic modulus, and the eighth elastic modulus is greater than the nineth elastic modulus. Thereby, the elastic modulus of the microcatheter can decrease gradually from the proximal-end segment to the connecting segment.

In addition, in the microcatheter of the present disclosure, optionally, a thickness of the first inner layer is less than a thickness of the first type polymer layer, and a thickness of the second inner layer is less than a thickness of the second type polymer layer.

In addition, in the microcatheter of the present disclosure, optionally, the first type polymer layer is made of one of a polyamide material, a polyether block polyamide material, a polyurethane material, an artificial rubber, and a synthetic rubber, and the second type polymer layer is made of one of a polyamide material, a polyether block polyamide material, a polyurethane material, an artificial rubber, or a synthetic rubber. Thereby, the first type polymer layer or the second type polymer layer can be made of one of the polyamide material, the polyether block polyamide material, the polyurethane material, the artificial rubber, and the synthetic rubber.

In addition, in the microcatheter according to the present disclosure, optionally, the first catheter body and the second catheter body are arranged side by side, and the first catheter body and the second catheter body are fixed thermoplastically by a protective tube sleeved thereon. Thereby, the process difficulty may be reduced.

In addition, in the microcatheter according to the present disclosure, optionally, the second catheter body includes a third type polymer layer, the third type polymer layer wraps and covers an outer side of the second type polymer layer, and the second type polymer layer and the third type polymer layer are made of different materials. Thereby, the stability of the microcatheter can be improved.

According to the present disclosure, it is able to provide a dual lumen microcatheter, with good push performance, flexibility, as well as excellent tensile resistance and bending resistance.

### BRIEF DESCRIPTION OF THE DRAWINGS

Embodiments of the present disclosure will now be explained in further detail, by way of example only, with reference to the accompanying drawings, in which:
Fig. 1 is a schematic diagram showing an overall structure of a dual lumen microcatheter according to an embodiment of the present disclosure.
Fig. 2 is a partially enlarged schematic diagram showing the dual lumen microcatheter according to an embodiment of the present disclosure.
Fig. 3 is a schematic diagram showing a layered catheter structure of a proximal-end segment of the dual lumen microcatheter according to an embodiment of the present disclosure.
Fig. 4 is a schematic diagram showing a cross-sectional structure of a catheter body taken along AA' in Fig. 2 according to the embodiment of the present disclosure.
Fig. 5 is a schematic diagram showing a cross-sectional structure taken along BB' in Fig. 2 according to an embodiment of the present disclosure.
Fig. 6 is a schematic diagram showing a cross-sectional structure taken along CC' in Fig. 2 according to an embodiment of the present disclosure.

### DETAILED DESCRIPTION

The present disclosure is described in further detail below with reference to the accompanying drawings and embodiments. In the drawings, identical components or functionally identical components are denoted by identical reference signs, and a repeated description thereof is omitted.

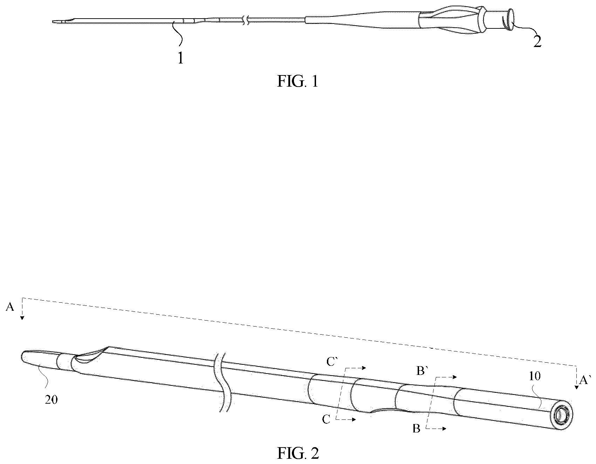

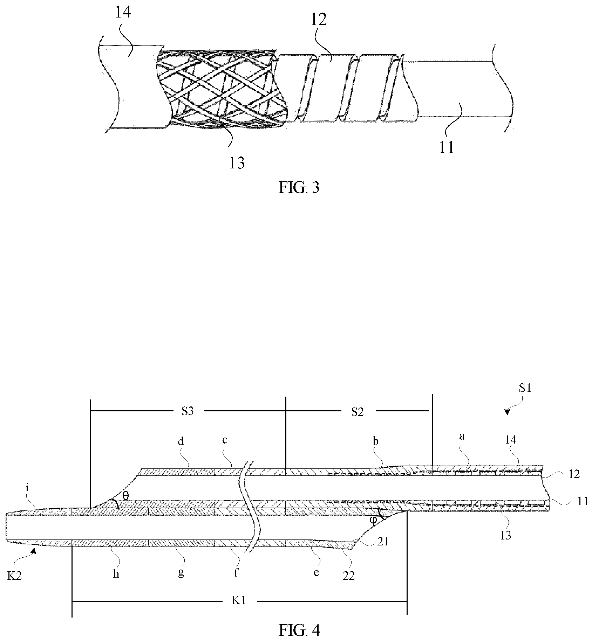

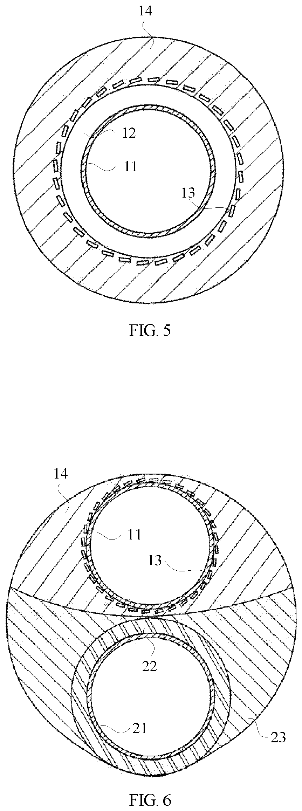

Fig. 1 is a schematic diagram showing an overall structure of a dual lumen microcatheter1 according to an embodiment of the present disclosure. Fig. 2 is a partially enlarged schematic diagram showing the dual lumen microcatheter1 according to an embodiment of the present disclosure. Fig. 3 is a schematic diagram showing a layered catheter structure of a proximal-end segment S1 of the dual lumen microcatheter 1 according to an embodiment of the present disclosure. Fig. 4 is a schematic diagram showing a cross-sectional structure of a catheter body taken along AA' in Fig. 2 according to the embodiment of the present disclosure. Fig. 5 is a schematic diagram showing a cross-sectional structure taken along BB' in Fig. 2 according to an embodiment of the present disclosure.

In this embodiment, the dual lumen microcatheter 1 according to the present disclosure may include a first catheter body 10 and a second catheter body 20. The second catheter body 20 may be connected to the first catheter body 10. The first catheter body 10 may include a first inner layer 11, a spring layer 12, a woven layer 13, and a polymer layer. At connection location of the first catheter body 10 and the second catheter body 20, the woven layer 13 in the first catheter body 10 may partially cover an outer side of the first inner layer 11 (see Figs. 1 to Fig. 4). In this case, the first inner layer 11, the spring layer 12, the woven layer 13, and the polymer layer of the first catheter body 10 are successively arranged from inside out, thereby the first catheter body 10 is facilitated to be advanced, and the joint of the first catheter body 10 and the second catheter body 20 has better flexibility. Thereby, a dual lumen microcatheter 1 with good push performance, flexibility, as well as excellent tensile resistance and bending resistance is provided.

In some examples, one end of the microcatheter 1 may be connected to a catheter base 2. In some examples, the catheter base 2 may allow a guide wire to pass therethrough.

In this embodiment, the interventional treatment of Chronic Total Occlusion (CTO) of the coronary artery includes: at first advancing the guide wire to the coronary arteryto reach a position where CTO of the coronary artery occurs, advancing the microcatheter 1 having the tip portion along the guide wire to the position where CTO of the coronary artery occurs, then advancing the guide wire into a lesion with the support force of the microcatheter 1, advancing the microcatheter 1 along the guide wire into the lesion, at last advancing the guide wire through a region where CTO of the coronary artery occurs with the support force of the microcatheter 1 having the tip portion. In the case of a bifurcation lesion or a lesion located at a blood vessel bifurcation, the guide wire may enter a false lumen and form a passage into the false lumen. After the guide wire enters the false lumen through a first guide wire inner lumen, the passage to the false lumen is blocked by the microcatheter 1, and the other guide wire is made to try to cross the lesion through a second guide wire inner lumen of the microcatheter 1 to enter a true lumen. In this case, since the passage to the false lumen is blocked by the microcatheter 1, the possibility of the second guide wire to enter the true lumen is increased very much.

In some examples, the microcatheter 1 may be advanced along with the guide wire in the procedure of the guide wire passing through the lesion. Thereby, it is able to continuously provide support force to the guide wire.

In other examples, the microcatheter 1 may be advanced alternately with the guide wire. Specifically, the microcatheter 1 maypass through the lesion region alternately with the guide wire. Thereby, the reliability of crossing can be improved, and continuous support force for the guide wire is provided.

In this embodiment, as described above, in some examples, the dual lumen microcatheter 1 may include the first catheter body 10 (see Fig. 2).

In this embodiment, the first catheter body 10 may have a proximal-end segment S1, a transition segment S2, and a distal-end segment S3 that are in sequential connection. In some examples, the distal-end segment S3 may have an inclined cut, i.e., an exit of the first guide wire inner lumen (described in more detail below). Specifically, an end of the distal-end segment S3 (i.e., the end of the distal-end segment S3 away from the proximal-end segment S1) has an inclined cut. Anoblique section of the inclined cut is curved. In some examples, the oblique section of the inclined cut of the distal-end segment S3 extends to the second catheter body 20 (described in more detail below). Specifically, the oblique section of the inclined cut of the distal-end segment S3 may extend to a second type polymer layer 22 (described in detail below) of the second catheter body 20. With reference to Fig. 4, the oblique section of the inclined cut of the distal-end segment S3 forms an acute angle θ with the second catheter body 20. In this case, the first catheter body 10 can be in size reduction gradually, thereby the flexibility of the microcatheter 1 can be gradually improvedas a whole.

In some examples, an outer diameter of the transition segment S2 may be decreased gradually from the proximal-end segment S1 to the distal-end segment S3, and pliability of the transition segment S2 is decreased gradually. Thereby, it is facilitated for the microcatheter 1 to pass through the blood vessel.

In this embodiment, the first catheter body 10 may include the first inner layer 11 (see Fig. 3 and Fig. 4). The first inner layer 11 may form the first guide wire inner lumen. That is, the first catheter body 10 may have the first guide wire inner lumen slidably receiving the guide wire. The first guide wire inner lumen extends from the proximal-end segment S1 to the inclined cut of the distal-end segment S3.

In some examples, in the first catheter body 10, the proximal-end segment S1 may have the first inner layer 11, the spring layer 12, the woven layer 13, and a first type polymer layer 14 (see Fig. 3 and Fig. 4). The first inner layer 11, the spring layer 12, the woven layer 13, and the first type polymer layer 14 may be arranged in a tight fit (see Fig. 5). Thereby, the tightness and reliability of the microcatheter 1 can be improved.

In some examples, as described above, the first inner layer 11 may form the first guide wire inner lumen. In the first catheter body 10, the proximal-end segment S1 may have the first inner layer 11 forming the first guide wire inner lumen (see Fig. 3).

In some examples, the first inner layer 11 may be made of one of polytetrafluoroethylene (PTFE), fluorinated ethylene propylene (FEP), perfluoroalkoxyalkane (PFA), polyethylene terephthalate (PET), and polyether ether ketone (PEEK).

In some examples, the spring layer 12 may be wound on the first inner layer 11 in winding manner and arranged along a length of the first inner layer 11 (see Fig. 3).

In some examples, the spring layer 12 may be made of metal, and in particular, the spring layer 12 may be made of 304 stainless steel wire.

In some examples, the woven layer 13 may be disposed on an outer side of the spring layer 12 (see Fig. 3 and Fig. 4).

In some examples, the woven layer 13 may be made of metal or fiber, and in particular, the woven layer 13 may be made of a 304 stainless steel material.

In some examples, the spring layer 12 may be formed with a wire spirally wound on the first inner layer 11. The woven layer 13 may be formed with a braid wire woven on the outer side of the spring layer 12. In this case, even if a continuous torque is input from the outside, the spring layer 12 can be protected from falling apart by the woven layer 13 on the outer side thereof, thereby improving the reliability of the microcatheter 1.

In some examples, the wire used for the spring layer 12 may be a flat wire. In some examples, the braid wire used for the woven layer 13 may be a flat wire. In some examples, the flat wire may be of a rectangular cross-section wire. Specifically, the spring layer 12 and the woven layer 13 may be wound on the first inner layer 11 with a wider side of the flat wire facing the first inner layer 11, resulting in reducing a thickness of the spring layer 12 and the woven layer 13. In addition, in some examples, the spring layer 12 may also use a round wire.

In other examples, a thickness of the flat wire used for spring layer 12 may be the same as a thickness of the flat wire used for woven layer 13. In addition, in some examples, the thickness of the wire used for the spring layer 12 may be greater than the thickness of the wire used for the woven layer 13. Thereby, the microcatheter 1 may obtain sufficient support force from the spring layer 12, and thus the push performance of the microcatheter 1 is facilitated.

In other examples, the thickness of the flat wire used for the spring layer 12 and the woven layer 13 may be uniform. In other examples, the thickness of the flat wire used for the spring layer 12 and the woven layer 13 may be non-uniform.

In some examples, the spring layer 12 may be arranged along a periphery of the first inner layer 11 and in tight fit against the first inner layer 11. For example, the spring layer 12 may be formed with wire wound clockwisely. In addition, the spring layer 12 may be formed with wire woundcounterclockwisely.

In some examples, the woven layer 13 may be arranged along the periphery of the first inner layer 11 and in tight fit against the spring layer 12. For example, the woven layer 13 may be formed with braid wire wound clockwisely. In addition, the woven layer 13 may be formed with the braid wire wound counterclockwisely. In other examples, the woven layer 13 may be formed with braid wires mesh staggered.

In some examples, the spring layer 12 is formed with wire spirally wound on the inner layer, and the woven layer 13 is formed withthe braid wire woven to the outer side of the spring layer 12. In this case, even if a continuous torque is input from the outside, the spring layer 12 can be protected from falling apart by the woven layer 13 on the outer side thereof, thereby improving the reliability of the microcatheter 1.

In some examples, as described above, in the first catheter body 10, the proximal-end segment S1 may have the first type polymer layer 14 (see Fig. 3 and Fig.4). The first type polymer layer 14 may cover an outer side of the woven layer 13 (see Fig. 3 and Fig. 4).

In some examples, the first type polymer layer 14 may be made of one of a polyamide material, a polyether block polyamide material, a polyurethane material, an artificial rubber, and a synthetic rubber.

In some examples, there may not be a well-defined boundary between the first inner layer 11 and the first type polymer layer 14. For example, the first inner layer 11 and the first type polymer layer 14 may be combined through welding brazing, thermoplastic processing, etc. In other examples, the first inner layer 11 and the first type polymer layer 14 may be made of the same material.

In some examples, a thickness of the first inner layer 11 may be less than a thickness of the first type polymer layer 14. A thickness of the second inner layer 21 (described in detail below) may be less than a thickness of the second type polymer layer 22 (described in detail below).

In some examples, a sum of the thickness of the spring layer 12 and the thickness of the woven layer 13 is less than the thickness of the first type polymer layer 14. Thereby, the push performance of the microcatheter is facilitated.

In this embodiment, in the first catheter body 10, the transition segment S2 may have the first inner layer 11, the woven layer 13, and the first type polymer layer 14 (see Fig. 4).

In some examples, the first inner layer 11 of the transition segment S2 may be formed with the first inner layer 11 of the proximal-end segment S1 extending to the transition segment S2. That is, the transition segment S2 may have the first inner layer 11 extending from the proximal-end segment S1.

In some examples, the woven layer 13 of the transition segment S2 may extend from the proximal-end segment S1 and be disposed on the outer side of the first inner layer 11.

In some examples, since the transition segment S2 does not include the spring layer 12, in this case, the woven layer 13 may be disposed directly on the outer side of the first inner layer 11 (see Fig. 4). That is, the woven layer 13 may be disposed on the outer side of the first inner layer 11 in a close fit.

In other examples, the woven layer 13 of the transition segment S2 may partially cover the outer side of the first inner layer 11.

In some examples, the first type polymer layer 14 of the transition segment S2 may extend from the proximal-end segment S1 and cover the outer side of the woven layer 13.

In some examples, the spring layer 12 does not extend to the transition segment S2 and the distal-end segment S3 (see Fig. 4). Specifically, the spring layer 12 is wound only on the outer side of the first inner layer 11 of the proximal-end segment S1, and the spring layer 12 stops winding or extending at a junction of the transition segment S2 and the proximal-end segment S1.

In this embodiment, in the first catheter body 10, the distal-end segment S3 may have the first inner layer 11 and the first type polymer layer 14 (see Fig. 4).

In some examples, the first inner layer 11 of the distal-end segment S3 may be formed with the first inner layer 11 of the transition segment S2 extending to the distal-end segment S3. That is, the distal-end segment S3 may have the first inner layer 11 extending from the transition segment S2.

In some examples, the first type polymer layer 14 of the distal-end segment S3 may extend from the transition segment S2 and cover the outer side of the first inner layer 11.

In some examples, in the first catheter body 10, the pliability of the proximal-end segment S1, the pliability of the transition segment S2, and the pliability of the distal-end segment S3 may decrease gradually. Thereby, the flexibility of the first catheter body 10 may be gradually improved.

In some examples, the first type polymer layer 14 may include a first polymer layer *a* that is disposed at the proximal-end segment S1 and has a first hardness, a second polymer layer *b* that is disposed at the transition segment S2 and has a second hardness, and a third polymer layer *c* that is disposed at the distal-end segment S3 and has a third hardness, and a fourth polymer layer *d* that is connected to the third polymer layer c and has a fourth hardness. In this case, the first type polymer layer 14 may be formed with a plurality of polymer layers combined, and the hardness of the different polymer layers may be different. Thereby, a suitable hardness may be selected according to different positions where the different polymer layers are located, further improving the bending resistance of the microcatheter 1 with flexibility ensured. Specifically, the polymer layers of different hardness may be sleeved outside the periphery of the first catheter body 10 in splicing manner, and then fixed to the periphery of the first catheter body 10 through hot-melting, thermoplastic processing, or heat-shrinking.

In some examples, the plurality of polymer layers in the first type polymer layer 14 may have different elastic modulus. Specifically, the first polymer layer *a* disposed at the proximal-end segment S 1 may have a first elastic modulus. The second polymer layer b disposed at the transition segment S2 may have a second elastic modulus. The third polymer layer c disposed at the distal-end segment S3 may have a third elastic modulus. The fourth polymer layer d connected to the third polymer layer c may have a fourth elastic modulus. In this case, the first type polymer layer 14 may be formed with the plurality of polymer layers with different elastic modulus combined. Thereby, it is able to select a suitable elastic modulus according to different positions where the different polymer layers are located, further improving the bending resistance of the microcatheter 1 while flexibility ensured. Specifically, the polymer layers with different elastic modulus may be sleeved outside the periphery of the first catheter body 10 in splicing manner, and then fixed to the periphery of the first catheter body 10 through hot-melting, thermoplastic processing or heat-shrinking.

In this embodiment, as described above, in some examples, the dual lumen microcatheter 1 may include the second catheter body 20 (see Fig. 2). The second catheter body 20 may be engaged to the periphery of the first catheter body 10 (see Fig. 4). In other words, the second catheter body 20 may partially contact the first catheter body 10.

In some examples, the second catheter body 20 may have a connecting segment K1 (see Fig. 4). The connecting segment K1 may be engaged to the periphery of the first catheter body 10. For example, the connecting segment K1 of the second catheter body 20 may contact the first catheter body 10.

In some examples, the second catheter body 20 may have a tip segment K2 (see Fig. 4). The tip segment K2 is engaged to the connecting segment K1 and is tapered.

In some examples, the first catheter body 10 does not contact the tip segment K2 of the second catheter body 20. In this case, the distal-end segment S3 of the first catheter body 10 can be connected to the connecting segment K1 of the second catheter body 20 without contacting the tip segment K2. Thereby, the tip segment K2 is able to extend further in a direction parallel to the distal-end segment S3.

In some examples, the connecting segment K1 of the second catheter body 20 is at least partially connected to the transition segment S2 of the first catheter body 10. Specifically, a projection of the connecting segment K1 at least partially in a radial direction coincides with a projection of the woven layer 13 in the radial direction.

In some examples, the connecting segment K1 and the tip segment K2 may be engaged to each other withanoblique section.

In some examples, an outer diameter of the connecting segment K1 may decrease gradually in a direction toward the tip of the tip segment K2, and the outer diameter of the tip segment K2 at anengaging portion thereof that is engaged to the connecting segment K1 is constant. Specifically, the outer diameter of the tip segment K2 at the engaging portion thereof that is engaged to the connecting segment K1 is consistent with the outer diameter of the connecting segment K1 at a position near the engaging portion. Thereby, the flexibility of the connecting segment K1 may be gradually changed to improve the bending resistance and tensile resistance. Wherein, the engaging portion of the tip segment K2 that is engaged to the connecting segment K1 may refer to a junction of the connecting segment K1 and the tip segment K2, as well as a portion near the junction.

In some examples, the gradual decrease of the outer diameter of the connecting segment K1 in the direction toward the tip of the tip segment K2 may be inuniform way. In other examples, the gradual decrease of the outer diameter of the connecting segment K1 in the direction toward the tip of the tip segment K2 may be in a way that the closer to the tip of the tip segment K2 the location is, the greater the decrease is. In addition, in some examples, the gradual decrease of the outer diameter of the connecting segment K1 in the direction toward the tip of the tip segment K2 may be in a way that the closer to the tip of the tip segment K2 the location is , the less the decrease is. In other examples, the gradual decrease of the outer diameter of the connecting segment K1 in the direction toward the tip of the tip segment K2 may be in a stepped manner. In this case, the manner of the decrease can be selected as desired, thereby different flexibilityis able to be obtained according to the manner of the decrease of the outer diameter of the connecting segment K1.

In some examples, the tip segment K2 may be tapered from the engaging portion. Specifically, the outer diameter of the tip segment K2 may decrease gradually in a direction away from the engaging portion.

In some examples, the connecting segment K1 may have an inclined cut, i.e., an entrance to the second guide wire inner lumen (described in detail below). Specifically, an end of the connecting segment K1 (i.e., the end of the connecting segment K1 away from the tip segment K2) has an inclined cut. Anoblique section of the inclined cut is curved. In some examples, the oblique section of the inclined cut of the connecting segment K1 extends to the first catheter body 10. Specifically, the oblique section of the inclined cut of the connecting segment K1 may extend tothe first type polymer layer 14 of the first catheter body 10. With reference to Fig. 4, the oblique section of the inclined cut of the connecting segment K1 forms an acute angle φ with the first catheter body 10. In this case, it is able to gradually increase the second catheter body 20, thereby reducing the difference in flexibility caused by the second catheter body 20 as a whole.

In this embodiment, the second catheter body 20 may have the second inner layer 21 (see Fig. 4). The second inner layer 21 may form the second guide wire inner lumen. That is, the second catheter body 20 may have the second guide wire inner lumen slidably receiving the guide wire. The second guide wire inner lumen extends from the connecting segment K1 to the tip segment K2.

In some examples, the second inner layer 21 may be made of one of polytetrafluoroethylene (PTFE), fluorinated ethylene propylene (FEP), perfluoroalkoxyalkane (PFA), polyethylene terephthalate (PET), and polyether ether ketone (PEEK).

In some examples, the second catheter body 20 may have the second type polymer layer 22 (see Fig. 4). The second type polymer layer 22 may cover the outer side of the second inner layer 21.

In some examples, the second type polymer layer 22 may be made of one of the polyamide material, the polyether block polyamide material, the polyurethane material, the artificial rubber, and the synthetic rubber.

In some examples, in the second catheter body 20, the connecting segment K1 may have the second inner layer 21 and the second type polymer layer 22 (see Fig. 4).

In some examples, in the second catheter body 20, the tip segment K2 may have the second inner layer 21 and the second type polymer layer 22 (see Fig. 4). In some examples, the material of the second type polymer layer 22 of the tip segment K2 may be a combination of resin and tungsten powder or a combination of resin and other developable materials. Thereby, it is facilitated to develop the second catheter body 20.

In some examples, as described above, the second inner layer 21 may form the second guide wire inner lumen. An inner diameter of the second guide wire inner lumen of the tip segment K2 may gradually decrease, and the decrease may be in uniform way. Thereby, the guide wire may be given greater support force. In other examples, the decrease of the inner diameter of the second guide wire inner lumen may be in a way that the closer to the tip the location is, the greater the decrease is.

In addition, in some examples, the decrease of the inner diameter of the second guide wire inner lumen may be in a way that the closer to the tip the location is, the less the decrease is. In other examples, the gradual decrease of the inner diameter of the second guide wire inner lumen may be in a stepper manner. In this case, the manner of the decrease can be selected as desired. Thereby, different properties are able to be obtained according to the manner of the decreaseof the inner diameter of the guide wire inner lumen.

In some examples, in the second catheter body 20, the pliability of the connecting segment K1 and the pliability of the tip segment K2 may decrease gradually. Thereby, it is able to gradually change the pliability of the microcatheter 1 to mitigate the risk of fracture, thereby improving the flexibility of the microcatheter 1 as a whole.

In some examples, the second type polymer layer 22 may include a fifth polymer layer e having a fifth hardness, a sixth polymer layer *f* connected to the fifth polymer layer *e* and having a sixth hardness, a seventh polymer layer g connected to the sixth polymer layer *f* and having a seventh hardness, an eighth polymer layer h connected to the seventh polymer layer g and having an eighth hardness, and a nineth polymer layer i having a nineth hardness. In this case, the second type polymer layer 22 may be formed with a plurality of polymer layers combined and form a cooperation with the first type polymer layer 14 at a connection location. Thereby, the flexibility of the microcatheter 1 can be adjusted with adjustment of the first type polymer layer 14 and the second type polymer layer 22. Specifically, the polymer layers with different hardness may be sleeved outside the periphery of the second catheter body 20 in splicing manner, and then fixed to the periphery of the second catheter body 20 through hot-melting, thermoplastic processing, or heat-shrinking.

In some examples, the plurality of polymer layers in the second type polymer layer 22 may have different elastic modulus. Specifically, the fifth polymer layer *e* may have a fifth elastic modulus. The sixth polymer layer *f* connected to the fifth polymer layer *e* may have a sixth elastic modulus. The seventh polymer layer g connected to the sixth polymer layer *f* may have a seventh elastic modulus. The eighth polymer layer h connected to the seventh polymer layer g may have an eighth elastic modulus. The nineth polymer layer i may have a nineth elastic modulus. In this case, the second type polymer layer 22 may be formed with a plurality of polymer layers combined and form a cooperation with the first type polymer layer 14 at the connection location. Thereby, the flexibility of the microcatheter 1 can be adjusted with adjustment of the first type polymer layer 14 and the second type polymer layer 22. Specifically, the polymer layers with different elastic modulus may be sleeved outside the periphery of the second catheter body 20 in splicing manner, and then fixed on the periphery of the second catheter body 20 through hot-melting, thermoplastic processing, or heat-shrinking.

In some examples, the first hardness may be greater than the fourth hardness, the third hardness may be equal to the sixth hardness, the fourth hardness may be equal to the seventh hardness, and the fifth hardness may be greater than the nineth hardness. Thereby, it is able to provide the microcatheter 1 with good flexibility, thereby improving the bending resistance and tensile resistance.

In some examples, the second hardness, the first hardness, the fifth hardness, the third hardness, the fourth hardness, the sixth hardness, the seventh hardness, the eighth hardness, and the nineth hardness may decrease gradually in sequence. Thereby, it is able to gradually change the hardness of the microcatheter 1 with stepped decrease in hardness while ensuring good flexibility.

In some examples, the second hardness, the first hardness, the fifth hardness, the third hardness, the fourth hardness, the eighth hardness, and the nineth hardness may decrease gradually in the order described. In this case, some of the polymer layers of the first catheter body 10 and the second catheter body 20 may have the same hardness. Thereby, flexibility difference in the polymer layers is able to be reduced, resulting in improving the bending resistance.

Further, specifically, the second hardness is greater than the first hardness, the first hardness is greater than the fifth hardness, the fifth hardness is greater than the third hardness, the third hardness is equal to the sixth hardness and is greater than the fourth hardness, the fourth hardness is equal to the seventh hardness and is greater than the eighth hardness, and the eighth hardness is greater than the nineth hardness.

In some examples, the overall elastic modulus of the microcatheter 1 decreases gradually from the proximal-end segment S1 to the tip segment K2. Thereby, the overall flexibility of the microcatheter 1 can be improved.

In some examples, the first elastic modulus may be greater than the fourth elastic modulus, the third elastic modulus may be equal to the sixth elastic modulus, the fourth elastic modulus may be equal to the seventh elastic modulus, and the fifth elastic modulus may be greater than the nineth elastic modulus. Thereby, it is able to provide the microcatheter 1 with good flexibility, thereby improving the bending resistance and tensile resistance.

In some examples, the second elastic modulus, the first elastic modulus, the fifth elastic modulus, the third elastic modulus, the fourth elastic modulus, the sixth elastic modulus, the seventh elastic modulus, the eighth elastic modulus, and the nineth elastic modulus may decrease gradually in sequence. Thereby, it is able to gradually change the elastic modulus of the microcatheter 1 with a stepped decrease in the elastic modulus while ensuring good flexibility.

In some examples, the second elastic modulus, the first elastic modulus, the fifth elastic modulus, the third elastic modulus, the fourth elastic modulus, the eighth elastic modulus, and the nineth elastic modulus may decrease gradually in the order described. In this case, some of the polymer layers of the first catheter body 10 and the second catheter body 20 may have the same elastic modulus. Thereby, flexibility difference in the polymer layers is able to be reduced, resulting in improving the bending resistance.

Further, specifically, the second elastic modulus is greater than the first elastic modulus, the first elastic modulus is greater than the fifth elastic modulus, the fifth elastic modulus is greater than the third elastic modulus, the third elastic modulus is equal to the sixth elastic modulus and greater than the fourth elastic modulus, the fourth elastic modulus is equal to the seventh elastic modulus and greater than the eighth elastic modulus, and the eighth elastic modulus is greater than the nineth elastic modulus. Thereby, the overall elastic modulus of the microcatheter 1 decreases gradually from the proximal-end segment S1 to the connecting segment K1.

Herein, a further description is provided below in connection with Fig. 4.

In some examples, for a comparison between the proximal-end segment S1 and the transition segment S2, the transition segment S2 lacks the spring layer 12; as a result, the first hardness of the first polymer layer *a* of the proximal-end segment S1 is less than the second hardness of the second polymer layer b of the transition segment S2, hence the flexibility of the transition segment S2 is lowered to an extent. However, as a whole, the flexibility of the transition segment S2 is still greater than the flexibility of the proximal-end segment S1. Moreover, the flexibility of the transition segment S2 is further gradually increased by reducing the thickness of the second polymer layer b of the transition segment S2 to a preset thickness.

In some examples, the fifth polymer layer *e* of the connecting segment K1 is connected to the second polymer layer b of the transition segment S2, resulting in increasing the thickness of the microcatheter 1 as a whole, hence the flexibility is reduced. Thus, the fifth hardness of the fifth polymer layer *e* is enabled to be smaller than the second hardness of the second polymer layer *b*, thereby offsetting a part of the impact on flexibility due to the second catheter body 20.

In some examples, the sixth hardness of the sixth polymer layer *f* of the connecting segment K1 is the same as the third hardness of the third polymer layer c of the distal-end segment S3, and the positions of the sixth polymer layer *f* and the third polymer layer c are the same, that is, their projections in the radial direction completely coincide. Thereby, the flexibility of the sixth polymer layer *f* and the third polymer layer c is stable, and good stability can be maintained even if the usage distance is extended.

In some examples, the seventh hardness of the seventh polymer layer g of the connecting segment K1 is the same as the fourth hardness of the fourth polymer layer d of the distal-end segment S3, and the seventh hardness is less than the sixth hardness, that is, the fourth hardness is less than the third hardness. Thereby, it is able to ensure that the change trend of flexibility is consistent and thus the overall stability of the microcatheter 1 is improved.

In some examples, the eighth hardness of the eighth polymer layer h of the connecting segment K1 is less than the seventh hardness of the seventh polymer layer g, and the eighth polymer layer *h* corresponds to an exit of the first catheter body 10 (i.e., the exit of the first guide wire inner lumen). Thereby, it is able to gradually increase the overall flexibility of the microcatheter 1. In addition, the nineth hardness of the nineth polymer layer i of the tip segment K2 is less than the eighth hardness of the eighth polymer layer *h.* Thereby, the eighth polymer layer *h* may serve as a transition between the connecting segment K1 and the tip segment K2, resulting in reducing the difference in flexibility and further improving the bending resistance of the microcatheter 1.

In some examples, polymer layers with different hardness may be made of different materials. In other examples, polymer layers with the same hardness may be made of the same material. However, the embodiment is not limited thereto, and the polymer layers with the same hardness may be made of different materials.

In the dual lumen microcatheter 1 according to the present disclosure, the guide wire can move through the first guide wire inner lumen of the first catheter body 10 and can also move through the second guide wire inner lumen of the second catheter body 20. The first catheter body 10 includes the proximal-end segment S1, a transition segment S2, and the distal-end segment S3, the proximal-end segment S1 has the inner layer, the spring layer 12, the woven layer 13, and the polymer layer, transition segment S2 has the inner layer, the woven layer 13, and the polymer layer partially covering the inner layer; the distal-end segment S3 has the inner layer and the polymer layer. The second catheter body 20 has the connecting segment K1 and the tip segment K2. In this case, the tapered tip segment K2 can facilitate the advancement of the microcatheter 1; the inner layer, the spring layer 12, the woven layer 13 and the polymer layer of the catheter body are arranged from inside to outside in sequence, as a result, the push performance of the catheter body is facilitated, and the connection of the first catheter body 10 and the second catheter body 20 has good flexibility and can form a good transition with the proximal-end segment S1. Thereby, the dual lumen microcatheter 1 with good push performance, flexibility, tensile resistance and bending resistance is provided.

Fig. 6 is a schematic diagram showing a cross-sectional structure taken along CC' in Fig. 2 according to an embodiment of the present disclosure.

In other examples, the first catheter body 10 and the second catheter body 20 are arranged side by side, and the first catheter body 10 and the second catheter body 20 are fixed thermoplasticallythrough a protective tube sleeved outside thereon. Thereby, the process difficulty can be reduced.

In some examples, the second catheter body 20 may include a third type polymer layer 23 (see Fig. 6). Wherein the third type polymer layer 23 may be wrapped on the outer side of the second type polymer layer 22. Thereby, the stability of the entire microcatheter 1 can be improved.

In some examples, the first type polymer layer 14, the second type polymer layer 22, and the third type polymer layer 23 may be made of different materials. In some examples, the second type polymer layer 22 and the third type polymer layer 23 may be made of the same material.

In some examples, the first type polymer layer 14 and the third type polymer layer 23 may be made of the same material. In this case, at the connection of the first catheter body 10 and the connecting segment K1, the first type polymer layer 14 and the third type polymer layer 23 are regarded as the first type polymer layer 14, and then the first type polymer layer 14 covers the second type polymer layer 22, that is, the first type polymer layer 14 wraps and covers the connecting segment K1. Thereby, it is able to improve the connectivity of the first catheter body 10 and the second catheter body 20, thereby improving the stability of the entire microcatheter 1.

In other examples, the tip segment K2 may have an inner tube and an outer tube. The outer tube may be disposed on an outer side of the inner tube. In some examples, the tip segment K2 is welded into engagement with the connecting segment K1 with the inner tube engaged to the connecting segment K1 and the outer tube sleevedoutsideand welded to the inner tube. Thereby, the push performance of the tip segment K2 is improved.

In other examples, the inner tube may extend from the outer tube along a length of the second catheter body 20. In this case, when the elastic modulus of the inner tube is less than that of the outer tube, the tip segment K2 can further reduce the elastic modulus of an end of the tip segment K2 away from the connection; when the elastic modulus of the inner tube is greater than that of the outer tube, the ability of the microcatheter 1 to pass through the lesion region can be increased; when the elastic modulus of the inner tube is equal to the elastic modulus of the outer tube, it is also able to reduce the elastic modulus of the end of the tip segment K2 away from the connection, thereby reducing the possibility of damage to the blood vessel.

In some examples, the elastic modulus of the outer tube is less than the elastic modulus of the connecting segment K1, and the elastic modulus of the inner tube is less than the elastic modulus of the connecting segment K1. Thereby, the overall elastic modulus of the tip segment K2 can be reduced, thereby improving the flexibility of the tip segment K2. In other examples, the elastic modulus of the tip segment K2 is less than the elastic modulus of the connecting segment K1. Thereby, the push performance and reliabilityof the dual lumen microcatheter 1 may be improved. In some examples, the outer tube may be tapered from an end near the connection to an end away from the connection. Thereby, the elastic modulus of the end of the tip segment K2 away from the connection can be reduced, thereby improving flexibility.

In some examples, the microcatheter 1 also includes a coating applied to inner walls of the first guide wire inner lumen and the second guide wire inner lumen. Thereby, it is facilitated for the guide wire to pass through the microcatheter 1. Specifically, the coating may be a highly lubricious hydrophilic polymer. In other examples, the coating may also be applied to outer walls of the first type polymer layer 14 and the second type polymer layer 22 of the microcatheter 1. Thereby, the friction can be reduced to facilitate advancement.

While the present disclosure is particularly shown and described with reference to the accompanying drawings and examples, it is to be understood that the disclosure is not limited in any way by the foregoing description. The scope of the invention is defined by the appended claims.

## Claims

1. A dual lumen microcatheter(1), comprising
a first catheter body (10) having a proximal-end segment (S1), a transition segment (S2), and a distal-end segment (S3) that are connected in sequence, and a second catheter body (20) engaged to a periphery of the first catheter body (10), the second catheter body (20) has a connecting segment (K1) engaged to the periphery of the first catheter body (10) and a tapered tip segment (K2) connected to the connecting segment (K1);
the first catheter body (10) has a first guide wire inner lumen slidably receiving a guide wire, and in the first catheter body (10), the proximal-end segment (S1) has a first inner layer (11) forming the first guide wire inner lumen, a spring layer (12) wound on the first inner layer (11) in winding manner and arranged along a length of the first inner layer (11), a woven layer (13) provided on an outer side of the spring layer (12), and a first type polymer layer (14) covering an outer side of the woven layer (13); the transition segment (S2) has the first inner layer (11) extending from the proximal-end segment (S1), a woven layer (13) extending from the proximal-end segment (S1) and partially covering an outer side of the first inner layer (11), and the first type polymer layer extending from the proximal-end segment (S1) and covering the outer side of the woven layer (13); the distal-end segment (S3) has the first inner layer (11) extending from the transition segment (S2) and the first type polymer layer (14) extending from the transition segment (S2) and covering the outer side of the first inner layer (11);
the second catheter body (20) has a second guide wire inner lumen slidably receiving a guide wire; the second catheter body (20) has a second inner layer (21) forming the second guide wire inner lumen and a second type polymer layer (22) covering an outer side of the second inner layer (21); the connecting segment (K1) of the second catheter body (20) is at least partially connected to the transition segment (S2) of the first catheter body (10).

2. The dual lumen microcatheter (1) according to claim 1, wherein
in the first catheter body (10), pliability of the proximal-end segment (S1), pliability of the transition segment (S2), and pliability of the distal-end segment (S3) decrease gradually, and in the second catheter body (20), pliability of the connecting segment (K1) and pliability of the tip segment (K2) decrease gradually.

3. The dual lumen microcatheter (1) according to claim 2, wherein wherein
the first type polymer layer (14) comprises a first polymer layer (a) disposed at the proximal-end segment (S1) and having a first hardness, a second polymer layer (b) disposed at the transition segment (S2) and having a second hardness, and a third polymer layer (c) disposed at the distal-end segment (S3) and having a third hardness, and a fourth polymer layer (d) connected to the third polymer layer (c) and having a fourth hardness;
the second type polymer layer (22) includes a fifth polymer layer (e) having a fifth hardness, a sixth polymer layer (f) connected to the fifth polymer layer (e) and having a sixth hardness, a seventh polymer layer (g) connected to the sixth polymer layer (f) and having a seventh hardness, an eighth polymer layer (h) connected to the seventh polymer layer (g) and having an eighth hardness, and a nineth polymer layer (i) having a nineth hardness.

4. The dual lumen microcatheter (1) according to claim 4, wherein
the first hardness is greater than the fourth hardness, the third hardness is equal to the sixth hardness, the fourth hardness is equal to the seventh hardness, and the fifth hardness is greater than the nineth hardness.

5. The dual lumen microcatheter (1) according to claim 4, wherein
the second hardness, the first hardness, the fifth hardness, the third hardness, the fourth hardness, the sixth hardness, the seventh hardness, the eighth hardness, and the nineth hardness decrease gradually in sequence.

6. The dual lumen microcatheter (1) according to claim 1, wherein
at a joint of the first catheter body (10) and the connecting segment (K1), the first type polymer layer (14) further wraps and covers the connecting segment (K1) such that the first type polymer layer (14) covers the second type polymer layer (22).

7. The dual lumen microcatheter (1) according to claim 1, wherein
the spring layer (12) is formed with a wire spirally wound on the first inner layer (11) in winding manner, and the woven layer (13) is formed with a braid wire woven on the outer side of the spring layer (12).

8. The dual lumen microcatheter (1) according to claim 1 or 2, wherein
the first type polymer layer (14) includes a first polymer layer (a) disposed at the proximal-end segment (S1) and having a first elastic modulus, a second polymer layer (b) disposed at the transition segment (S2) and having a second elastic modulus, a third polymer layer (c) disposed at the distal-end segment (S3) and having a third elastic modulus, and a fourth polymer layer (d) connected to the third polymer layer (c) and having a fourth elastic modulus;
the second type polymer layer (22) includes a fifth polymer layer (e) having a fifth elastic modulus, a sixth polymer layer (f) connected to the fifth polymer layer (e) and having a sixth elastic modulus, a seventh polymer layer (g) connected to the sixth polymer layer (f) and having a seventh elastic modulus, an eighth polymer layer (h) connected to the seventh polymer layer (g) and having an eighth elastic modulus, and a nineth polymer layer (i) having a nineth elastic modulus.

9. The dual lumen microcatheter (1) according to claim 1 or 2 wherein
the first elastic modulus is greater than the fourth elastic modulus, the third elastic modulus is equal to the sixth elastic modulus, the fourth elastic modulus is equal to the seventh elastic modulus, and the fifth elastic modulus is greater than the nineth elastic modulus.

10. The dual lumen microcatheter (1) according to claim 9, wherein
the second elastic modulus, the first elastic modulus, the fifth elastic modulus, the third elastic modulus, the fourth elastic modulus, the eighth elastic modulus, and the nineth elastic modulus decrease gradually in sequence.

11. The dual lumen microcatheter (1) according to claim 9, wherein
the second elastic modulus is greater than the first elastic modulus, the first elastic modulus is greater than the fifth elastic modulus, the fifth elastic modulus is greater than the third elastic modulus, the third elastic modulus is equal to the sixth elastic modulus and greater than the fourth elastic modulus, the fourth elastic modulus is equal to the seventhelastic modulus and greater than the eighth elastic modulus, and the eighth elastic modulus is greater than the nineth elastic modulus.

12. The dual lumen microcatheter (1) according to claim 1, wherein
a thickness of the first inner layer (11) is less than a thickness of the first type polymer layer (14), and a thickness of the second inner layer (21) is less than a thickness of the second type polymer layer (22).

13. The dual lumen microcatheter (1) according to claim 1, wherein
the first type polymer layer (14) is made of one of a polyamide material, a polyether block polyamide material, a polyurethane material, an artificial rubber, and a synthetic rubber, and the second type polymer layer (22) is made of one of a polyamide material, a polyether block polyamide material, a polyurethane material, an artificial rubber, and a synthetic rubber.

14. The dual lumen microcatheter (1) according to claim 1, wherein
the first catheter body (10) and the second catheter body (20) are arranged side by side, and the first catheter body (10) and the second catheter body (20) are fixed thermoplasticallywith a protective tube sleeved outside thereon.

15. The dual lumen microcatheter (1) according to claim 1, wherein the second catheter body (20) includes a third type polymer layer (23), the third type polymer layer (23) wraps and covers an outer side of the second type polymer layer (22), and the second type polymer layer (22) and the third type polymer layer (23) are made of different materials.

## Patentansprüche

1. Doppellumen-Mikrokatheter(1), Folgendes umfassend
einen ersten Katheterkörper (10), der ein proximales Endsegment (S1), ein Übergangssegment (S2) und ein distales Endsegment (S3) aufweist, die der Reihe nach miteinander verbunden sind, und einen zweiten Katheterkörper (20), der mit einer Peripherie des ersten Katheterkörpers (10) in Eingriff steht, wobei der zweite Katheterkörper (20) ein Verbindungssegment (K1), das mit der Peripherie des ersten Katheterkörpers (10) in Eingriff steht, und ein verjüngtes Spitzensegment (K2) aufweist, das mit dem Verbindungssegment (K1) verbunden ist;
wobei der erste Katheterkörper (10) ein erstes Führungsdraht-Innenlumen aufweist, das gleitend einen Führungsdraht aufnimmt, und in dem ersten Katheterkörper (10) das proximale Endsegment (S1) eine erste Innenschicht (11), die das erste Führungsdraht-Innenlumen bildet, eine Federschicht (12), die auf die erste Innenschicht (11) in gewundener Weise gewickelt und entlang einer Länge der ersten Innenschicht (11) angeordnet ist, eine gewebte Schicht (13), die auf einer Außenseite der Federschicht (12) vorgesehen ist, und eine Polymerschicht des ersten Typs (14), die eine Außenseite der gewebten Schicht (13) bedeckt, aufweist; das Übergangssegment (S2) die erste Innenschicht (11), die sich von dem proximalen Endsegment (S1) erstreckt, eine gewebte Schicht (13), die sich von dem proximalen Endsegment (S1) erstreckt und teilweise eine Außenseite der ersten Innenschicht (11) bedeckt, und die Polymerschicht des ersten Typs aufweist, die sich von dem proximalen Endsegment (S1) erstreckt und die Außenseite der gewebten Schicht (13) bedeckt; das distale Endsegment (S3) die erste Innenschicht (11), die sich von dem Übergangssegment (S2) erstreckt, und die Polymerschicht (14) des ersten Typs aufweist, die sich von dem Übergangssegment (S2) erstreckt und die Außenseite der ersten Innenschicht (11) bedeckt;
der zweite Katheterkörper (20) ein zweites Führungsdraht-Innenlumen aufweist, das gleitend einen Führungsdraht aufnimmt; der zweite Katheterkörper (20) eine zweite Innenschicht (21), die das zweite Führungsdraht-Innenlumen bildet, und eine Polymerschicht des zweiten Typs (22) aufweist, die eine Außenseite der zweiten Innenschicht (21) bedeckt; das Verbindungssegment (K1) des zweiten Katheterkörpers (20) mindestens teilweise mit dem Übergangssegment (S2) des ersten Katheterkörpers (10) verbunden ist.

2. Doppellumen-Mikrokatheter(1) nach Anspruch 1, wobei
in dem ersten Katheterkörper (10) die Biegsamkeit des proximalen Endsegments (S1), die Biegsamkeit des Übergangssegments (S2) und die Biegsamkeit des distalen Endsegments (S3) allmählich abnehmen und in dem zweiten Katheterkörper (20) die Biegsamkeit des Verbindungssegments (K1) und die Biegsamkeit des Spitzensegments (K2) allmählich abnehmen.

3. Doppellumen-Mikrokatheter(1) nach Anspruch 2, wobei
die Polymerschicht des ersten Typs (14) eine erste Polymerschicht (a), die an dem proximalen Endsegment (S1) angeordnet ist und eine erste Härte aufweist, eine zweite Polymerschicht (b), die an dem Übergangssegment (S2) angeordnet ist und eine zweite Härte aufweist, und eine dritte Polymerschicht (c), die an dem distalen Endsegment (S3) angeordnet ist und eine dritte Härte aufweist, und eine vierte Polymerschicht (d) umfasst, die mit der dritten Polymerschicht (c) verbunden ist und eine vierte Härte aufweist;
die Polymerschicht des zweiten Typs (22) eine fünfte Polymerschicht (e), die eine fünfte Härte aufweist, eine sechste Polymerschicht (f), die mit der fünften Polymerschicht (e) verbunden ist und eine sechste Härte aufweist, eine siebte Polymerschicht (g), die mit der sechsten Polymerschicht (f) verbunden ist, und eine siebte Härte aufweist, eine achte Polymerschicht (h), die mit der siebten Polymerschicht (g) verbunden ist, und eine achte Härte aufweist und eine neunte Polymerschicht (i) umfasst, die eine neunte Härte aufweist.

4. Doppellumen-Mikrokatheter(1) nach Anspruch 4, wobei
die erste Härte größer als die vierte Härte ist, die dritte Härte gleich der sechsten Härte ist, die vierte Härte gleich der siebten Härte ist und die fünfte Härte größer als die neunte Härte ist.

5. Doppellumen-Mikrokatheter(1) nach Anspruch 4, wobei
die zweite Härte, die erste Härte, die fünfte Härte, die dritte Härte, die vierte Härte, die sechste Härte, die siebte Härte, die achte Härte und die neunte Härte der Reihe nach abnehmen.

6. Doppellumen-Mikrokatheter(1) nach Anspruch 1, wobei
an einer Verbindung des ersten Katheterkörpers (10) und des Verbindungssegments (K1) die Polymerschicht des ersten Typs (14) das Verbindungssegment (K1) weiter umhüllt und bedeckt, sodass die Polymerschicht des ersten Typs (14) die Polymerschicht des zweiten Typs (22) bedeckt.

7. Doppellumen-Mikrokatheter(1) nach Anspruch 1, wobei
die Federschicht (12) mit einem spiralförmig auf die erste Innenschicht (11) gewickelten Draht gebildet ist, und die gewebte Schicht (13) mit einem auf der Außenseite der Federschicht (12) gewebten Flechtdraht gebildet ist.

8. Doppellumen-Mikrokatheter(1) nach Anspruch 1 oder 2, wobei
die Polymerschicht des ersten Typs (14) eine erste Polymerschicht (a), die an dem proximalen Endsegment (S1) angeordnet ist und einen ersten Elastizitätsmodul aufweist, eine zweite Polymerschicht (b), die an dem Übergangssegment (S2) angeordnet ist und einen zweiten Elastizitätsmodul aufweist, eine dritte Polymerschicht (c), die an dem distalen Endsegment (S3) angeordnet ist und einen dritten Elastizitätsmodul aufweist, und eine vierte Polymerschicht (d) umfasst, die mit der dritten Polymerschicht (c) verbunden ist und einen vierten Elastizitätsmodul aufweist;
die Polymerschicht des zweiten Typs (22) eine fünfte Polymerschicht (e), die einen fünften Elastizitätsmodul aufweist, eine sechste Polymerschicht (f), die mit der fünften Polymerschicht (e) verbunden ist und einen sechsten Elastizitätsmodul aufweist, eine siebte Polymerschicht (g), die mit der sechsten Polymerschicht (f) verbunden ist und einen siebten Elastizitätsmodul aufweist, eine achte Polymerschicht (h), die mit der siebten Polymerschicht (g) verbunden ist und einen achten Elastizitätsmodul aufweist, und eine neunte Polymerschicht (i) umfasst, die einen neunten Elastizitätsmodul aufweist.

9. Doppellumen-Mikrokatheter (1) nach Anspruch 1 oder 2, wobei
der erste Elastizitätsmodul größer als der vierte Elastizitätsmodul ist, der dritte Elastizitätsmodul gleich dem sechsten Elastizitätsmodul ist, der vierte Elastizitätsmodul gleich dem siebten Elastizitätsmodul ist und der fünfte Elastizitätsmodul größer als der neunte Elastizitätsmodul ist.

10. Doppellumen-Mikrokatheter (1) nach Anspruch 9, wobei
der zweite Elastizitätsmodul, der erste Elastizitätsmodul, der fünfte Elastizitätsmodul, der dritte Elastizitätsmodul, der vierte Elastizitätsmodul, der achte Elastizitätsmodul und der neunte Elastizitätsmodul allmählich der Reihe nach abnehmen.

11. Doppellumen-Mikrokatheter (1) nach Anspruch 9, wobei
der zweite Elastizitätsmodul größer als der erste Elastizitätsmodul ist, der erste Elastizitätsmodul größer als der fünfte Elastizitätsmodul ist, der fünfte Elastizitätsmodul größer als der dritte Elastizitätsmodul ist, der dritte Elastizitätsmodul gleich dem sechsten Elastizitätsmodul und größer als der vierte Elastizitätsmodul ist, der vierte Elastizitätsmodul gleich dem siebten Elastizitätsmodul und größer als der achte Elastizitätsmodul ist und der achte Elastizitätsmodul größer als der neunte Elastizitätsmodul ist.

12. Doppellumen-Mikrokatheter (1) nach Anspruch 1, wobei
eine Dicke der ersten inneren Schicht (11) geringer ist als eine Dicke der Polymerschicht des ersten Typs (14) und eine Dicke der zweiten inneren Schicht (21) geringer ist als eine Dicke der Polymerschicht des zweiten Typs (22).

13. Doppellumen-Mikrokatheter (1) nach Anspruch 1, wobei
die Polymerschicht des ersten Typs (14) aus einem von einem Polyamidmaterial, einem Polyether-Block-PolyamidMaterial, einem Polyurethanmaterial, einem künstlichen Kautschuk und einem synthetischen Kautschuk hergestellt ist, und die Polymerschicht des zweiten Typs (22) aus einem von einem Polyamidmaterial, einem Polyether-Block-Polyamid-Material, einem Polyurethanmaterial, einem künstlichen Kautschuk und einem synthetischen Kautschuk hergestellt ist.

14. Doppellumen-Mikrokatheter (1) nach Anspruch 1, wobei
der erste Katheterkörper (10) und der zweite Katheterkörper (20) nebeneinander angeordnet sind und der erste Katheterkörper (10) und der zweite Katheterkörper (20) thermoplastisch mit einem außen ummantelten Schutzschlauch befestigt sind.

15. Doppellumen-Mikrokatheter (1) nach Anspruch 1, wobei
der zweite Katheterkörper (20) eine Polymerschicht des dritten Typs (23) beinhaltet, die Polymerschicht des dritten Typs (23) eine Außenseite der Polymerschicht des zweiten Typs (22) umhüllt und bedeckt, und die Polymerschicht des zweiten Typs (22) und die Polymerschicht des dritten Typs (23) aus unterschiedlichen Materialien hergestellt sind.

## Revendications

1. Microcathéter à double lumière (1), comprenant
un premier corps de cathéter (10) ayant un segment d'extrémité proximale (S1), un segment de transition (S2) et un segment d'extrémité distale (S3) qui sont reliés en séquence, et un deuxième corps de cathéter (20) en prise avec une périphérie du premier corps de cathéter (10), le deuxième corps de cathéter (20) a un segment de liaison (K1) en prise avec la périphérie du premier corps de cathéter (10) et un segment à pointe conique (K2) raccordé au segment de liaison (K1) ;
le premier corps de cathéter (10) a une première lumière interne de fil-guide recevant de manière coulissante un fil-guide, et dans le premier corps de cathéter (10), le segment d'extrémité proximale (S1) a une première couche interne (11) formant la première lumière interne du fil-guide, une couche de ressort (12) enroulée sur la première couche interne (11) de manière enroulée et agencée le long d'une longueur de la première couche interne (11), une couche tissée (13) disposée sur un côté externe de la couche à ressort (12), et une couche de polymère de premier type (14) recouvrant un côté externe de la couche tissée (13) ; le segment de transition (S2) a la première couche interne (11) s'étendant à partir du segment d'extrémité proximale (S1), une couche tissée (13) s'étendant à partir du segment d'extrémité proximale (S1) et recouvrant partiellement un côté externe de la première couche interne (11), et la couche de polymère de premier type s'étendant à partir du segment d'extrémité proximale (S1) et recouvrant le côté externe de la couche tissée (13) ; le segment d'extrémité distale (S3) a la première couche interne (11) s'étendant à partir du segment de transition (S2) et la couche de polymère de premier type (14) s'étendant à partir du segment de transition (S2) et recouvrant le côté externe de la première couche interne (11) ;
le deuxième corps de cathéter (20) a une deuxième lumière interne de fil-guide recevant de manière coulissante un fil-guide ; le deuxième corps de cathéter (20) a une deuxième couche interne (21) formant la deuxième lumière interne du fil-guide et une couche de polymère de deuxième type (22) recouvrant un côté externe de la deuxième couche interne (21) ; le segment de liaison (K1) du deuxième corps de cathéter (20) est au moins partiellement relié au segment de transition (S2) du premier corps de cathéter (10).

2. Microcathéter à double lumière (1) selon la revendication 1, dans lequel
dans le premier corps de cathéter (10), la pliabilité du segment d'extrémité proximale (S1), la pliabilité du segment de transition (S2), et la pliabilité du segment d'extrémité distale (S3) diminuent progressivement, et dans le deuxième corps de cathéter (20), la pliabilité du segment de liaison (K1) et la pliabilité du segment de pointe (K2) diminuent progressivement.

3. Microcathéter à double lumière (1) selon la revendication 2, dans lequel dans lequel
la couche de polymère de premier type (14) comprend une première couche de polymère (a) disposée au niveau du segment d'extrémité proximale (S1) et ayant une première dureté, une deuxième couche de polymère (b) disposée au niveau du segment de transition (S2) et ayant une deuxième dureté, et une troisième couche de polymère (c) disposée au niveau du segment d'extrémité distale (S3) et ayant une troisième dureté, et une quatrième couche de polymère (d) reliée à la troisième couche de polymère (c) et ayant une quatrième dureté ;
la couche de polymère de deuxième type (22) comprend une cinquième couche de polymère (e) ayant une cinquième dureté, une sixième couche de polymère (f) reliée à la cinquième couche de polymère (e) et ayant une sixième dureté, une septième couche de polymère (g) reliée à la sixième couche de polymère (f) et ayant une septième dureté, une huitième couche de polymère (h) reliée à la septième couche de polymère (g) et ayant une huitième dureté, et une neuvième couche de polymère (i) ayant une neuvième dureté.

4. Microcathéter à double lumière (1) selon la revendication 4, dans lequel
la première dureté est supérieure à la quatrième dureté, la troisième dureté est égale à la sixième dureté, la quatrième dureté est égale à la septième dureté et la cinquième dureté est supérieure à la neuvième dureté.

5. Microcathéter à double lumière (1) selon la revendication 4, dans lequel
la deuxième dureté, la première dureté, la cinquième dureté, la troisième dureté, la quatrième dureté, la sixième dureté, la septième dureté, la huitième dureté, et la neuvième dureté diminuent progressivement en séquence.

6. Microcathéter à double lumière (1) selon la revendication 1, dans lequel
au niveau d'une jonction du premier corps de cathéter (10) et du segment de liaison (K1), la couche de polymère de premier type (14) enveloppe et recouvre en outre le segment de liaison (K1) de telle sorte que la couche de polymère de premier type (14) recouvre la couche de polymère de deuxième type (22).

7. Microcathéter à double lumière (1) selon la revendication 1, dans lequel
la couche de ressort (12) est formée avec un fil enroulé en spirale sur la première couche interne (11) de manière enroulée, et la couche tissée (13) est formée avec un fil tressé tissé sur le côté externe de la couche de ressort (12).

8. Microcathéter à double lumière (1) selon la revendication 1 ou 2, dans lequel
la couche de polymère de premier type (14) comporte une première couche de polymère (a) disposée au niveau du segment d'extrémité proximale (S1) et ayant un premier module d'élasticité, une deuxième couche de polymère (b) disposée au niveau du segment de transition (S2) et ayant un deuxième module d'élasticité, une troisième couche de polymère (c) disposée au niveau du segment d'extrémité distale (S3) et ayant un troisième module d'élasticité, et une quatrième couche de polymère (d) reliée à la troisième couche de polymère (c) et ayant un quatrième module d'élasticité ;
la couche de polymère de deuxième type (22) comprend une cinquième couche de polymère (e) ayant un cinquième module d'élasticité, une sixième couche de polymère (f) reliée à la cinquième couche de polymère (e) et ayant un sixième module d'élasticité, une septième couche de polymère (g) reliée à la sixième couche de polymère (f) et ayant un septième module d'élasticité, une huitième couche de polymère (h) reliée à la septième couche de polymère (g) et ayant un huitième module d'élasticité, et une neuvième couche de polymère (i) ayant un neuvième module d'élasticité

9. Microcathéter à double lumière (1) selon la revendication 1 ou 2 dans lequel
le premier module d'élasticité est supérieur au quatrième module d'élasticité, le troisième module d'élasticité est égal au sixième module d'élasticité, le quatrième module d'élasticité est égal au septième module d'élasticité et le cinquième module d'élasticité est supérieur au neuvième module d'élasticité

10. Microcathéter à double lumière (1) selon la revendication 9, dans lequel
le deuxième module d'élasticité, le premier module d'élasticité, le cinquième module d'élasticité, le troisième module d'élasticité, le quatrième module d'élasticité, le huitième module d'élasticité et le neuvième module d'élasticité diminuent progressivement en séquence.

11. Microcathéter à double lumière (1) selon la revendication 9, dans lequel
le deuxième module d'élasticité est supérieur au premier module d'élasticité, le premier module d'élasticité est supérieur au cinquième module d'élasticité, le cinquième module d'élasticité est supérieur au troisième module d'élasticité, le troisième module d'élasticité est égal au sixième module d'élasticité et supérieur au quatrième module d'élasticité, le quatrième module d'élasticité est égal au septième module d'élasticité et supérieur au huitième module d'élasticité, et le huitième module d'élasticité est supérieur au neuvième module d'élasticité

12. Microcathéter à double lumière (1) selon la revendication 1, dans lequel
une épaisseur de la première couche interne (11) est inférieure à une épaisseur de la couche de polymère de premier type (14), et une épaisseur de la deuxième couche interne (21) est inférieure à une épaisseur de la couche de polymère de deuxième type (22).

13. Microcathéter à double lumière (1) selon la revendication 1, dans lequel
la couche de polymère de premier type (14) est constituée de l'un parmi un matériau polyamide, un matériau polyamide à blocs polyéthers, un matériau en polyuréthane, un caoutchouc artificiel et un caoutchouc synthétique, et la couche de polymère du deuxième type (22) est constituée d'un matériau polyamide, un matériau polyamide à blocs polyéthers, un matériau en polyuréthane, un caoutchouc artificiel, et un caoutchouc synthétique.

14. Microcathéter à double lumière (1) selon la revendication 1, dans lequel
le premier corps de cathéter (10) et le deuxième corps de cathéter (20) sont agencés côte à côte, et le premier corps de cathéter (10) et le deuxième corps de cathéter (20) sont fixés de manière thermoplastique avec un tube protecteur manchonné à l'extérieur de celui-ci.

15. Microcathéter à double lumière (1) selon la revendication 1, dans lequel
le deuxième corps de cathéter (20) comprend une couche de polymère de troisième type (23), la couche de polymère de troisième type (23) enveloppe et recouvre un côté externe de la couche de polymère de deuxième type (22), et la couche de polymère de deuxième type (22) et la couche de polymère de troisième type (23) sont constituées de matériaux différents.
